# EUROPEAN PATENT APPLICATION

(11) **EP 0 613 873 A2**
(43) Date of publication of application: **07.09.1994**
(21) Application number: 94200431.8
(22) Date of filing: 21.02.1994
(51) Int. Cl.: C07C 2/34

(54) **Oligomerisation process**

(30) Priority: 23.02.1993 EP 93301335
(71) Applicant: SHELL INTERNATIONALE RESEARCH MAATSCHAPPIJ B.V., NL-2596 HR Den Haag (NL)
(72) Inventor: Van Zon, Arie, NL-1031 CM Amsterdam (NL); De Boer, Eric Johannes Maria, NL-1031 CM Amsterdam (NL); De Boer,Henricus Jacobus Robert, NL-1031 CM Amsterdam (NL)

(57) **Abstract**

The invention provides a process for the preparation of liquid organic compounds, suitable as base materials for lubricants, comprising contacting one of more alpha-olefins containing 8 to 20 carbon atoms per molecule, under oligomerisation conditions with a catalyst composition based on
a) a Group IV A metal compound of the general formula (Cp)₂ MeX₂, wherein Cp represents a cyclopentadienyl group, Me represents a Group IV A metal and each X independently represents a moiety selected from the group consisting of hydrocarbyl groups, hydrocarboxy groups, hydrocarbamido groups, each of which may be optionally substituted, hydrogen atoms and halogen atoms and
b) a substantially non-coordinating anion source;
   and optionally subjecting the oligomers formed to an addition reaction to reduce their olefinic unsaturation.

## Description

The invention relates to a process for preparing liquid organic compounds, suitable as base materials for lubricants, which process involves a catalytic oligomerisation reaction.

It is known that oligomers of propylene may be used as base materials for preparing lubricating oils. As is apparent from some earlier publications, propylene oligomers with a polymerisation degree of mainly 2 to 10 and containing vinyl-end groups, have been considered of particular interest. For example, in EP-A-268 214, propylene oligomers having a polymerisation degree of mainly 2 to 10 and containing vinyl end-groups are prepared in high selectivity by oligomerising propylene in the presence of a catalyst comprising a transition metal compound and an organometallic compound, wherein an alkyl-substituted cyclopentadienyl compound of zirconium and/or an alkyl-substituted cyclopentadienyl compound of hafnium is used as the transition metal compound, and a condensation product of an organoaluminium compound and water is used as the organometallic compound.

The transition metal compound may be represented by the formula

(R₅C₅)ₘ M X₄₋ₘ (I)

wherein R represents a C₁-C₂₀ alkyl group, R₅C₅ represents a cyclopentadienyl group substituted with an alkyl group, M represents a zirconium or hafnium atom, X represents a hydrogen or halogen atom, or a C₁-C₂₀ alkyl group, and m represents an integer from 2 to 4.

The organometallic compound is exemplified by methylaluminoxane, ethylaluminoxane, propylaluminoxane, isopropylaluminoxane, butylaluminoxane, isobutylaluminoxane and amylaluminoxane.

In Table 1 on Page 8 of EP-A-268 214, the results are shown of a comparative experiment (Comparative Example 1) in which the catalyst used contained an unsubstituted cyclopentadienyl zirconium compound, i.e. bis(cyclopentadienyl)zirconium dichloride, as the transition metal compound and methylaluminoxane as the organometallic compound. It will be seen from Table 1 that a polymerisation degree of 41.9 was obtained using this catalyst. Although no data is given on the selectivity of the catalyst with respect to vinyl and vinylidene end-groups (in contrast to the catalysts of Examples 1 to 18 which contained a transition metal compound of formula I above), it is stated in the text at Page 7, lines 6 to 8 that in the comparative examples, polymerisation reaction took place preferentially to oligomerisation reaction and product were all high polymers which predominantly had vinylidene group as terminal unsaturated group.

There is no further structural information provided in EP-A-268 214 on the comparison products.

However, in Examples 1 to 4 of EP-A-490 454, atactic propylene oligomers having number average molecular weights 1070, 1455, 1710 and 2130 and thus polymerisation degrees respectively of 25.5, 34.6, 40.7 and 50.7, were prepared according to methods analogous to that disclosed in Comparative Example 1 of EP-A-268 214, but using a higher molar ratio of methylaluminoxane to bis(cyclopentadienyl)zirconium dichloride. The molecular structures of the oligomers were investigated by ¹³C nuclear magnetic resonance spectroscopy and were found to be substantially of the formula, 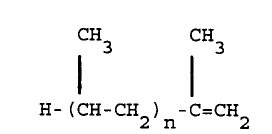
with at least 95% of polymer chains containing a vinylidene end-group.

In EP-A-69 951, a process is disclosed for the preparation of a polyolefin by polymerising an olefin of the formula CH₂CHR in which R is hydrogen or C₁-C₁₀ alkyl, on its own or as a mixture, if appropriate as a mixture with C₄-C₁₂ alpha, omega diolefins, in solvents, liquid monomers or the gas phase, at temperatures between -50 and 200°C, using a soluble, halogen-containing transition metal compound and aluminoxanes, which comprises carrying out the polymerisation in the presence of a catalyst system composed of the following components:
a) a transition metal compound of the general formula
   (Cyclopentadienyl)₂ Me R Hal
   in which R is a cyclopentadienyl or C₁-C₆ alkyl group, or a halogen atom, especially chlorine, Me is a transition metal, especially zirconium, and Hal is a halogen atom, especially chlorine,
b) an aluminium compound of the aluminoxane type having the general formulae

   Al₂OR₄(Al(R)-O)ₙ

   for a linear aluminoxane, and

   (Al(R)-O)ₙ₊₂

   for a cyclic aluminoxane, in which n is an integer from 4 to 20, and R is a methyl or ethyl group, preferably a methyl group.

Preferred catalyst systems are those consisting of bis(cyclopentadienyl)zirconium dichloride or bis(cyclopentadienyl)zirconium monomethylmonochloride and methylaluminoxane.

The catalyst system of EP-A-69 951 is said to be advantageous when compared to halogen-free catalyst systems in that a higher polymerisation activity is observed at useful polymerisation temperatures between 40 and 80°C. It is also stated to be advantageous compared to other halogen-containing catalyst systems, e.g. Ziegler catalysts, in that it contains proportionately less halogen resulting in, not only the formation of polyolefins having a lower halogen content (which is beneficial from an environmental standpoint), but also less corrosion of the reactor.

In the examples of EP-A-69 951, the preparation of polymers from ethylene (Examples 1, 2 and 3), propylene (Example 4), 1-hexene (Example 6) and mixtures of ethylene with 1-butene (Example 5) or with 1-hexene (Example 7) are described. The number average molecular weights of the polymers obtained, where given, are generally high. Indeed, the ethylene polymers of Examples 1 and 2 had number average molecular weights respectively of 91,000 and 1,000,000, and the propylene polymer of Example 4 had number average molecular weight of 5,000.

Although it is stated in the text at Page 3, lines 31 and 32 that the transition metal and aluminium are used in an atomic ratio of 10:1 to 10⁸:1, it is clear from the examples of the specification that this statement is incorrect. For example, if reference is made to Example 6 describing the preparation of the 1-hexene polymer, it will be seen that the catalyst system comprised 130 mg methylaluminoxane (corresponding to 2.2 mmol aluminium) and 6.66 x 10⁻⁷ mol bis(cyclopentadienyl)zirconium dichloride (corresponding to 6.66 x 10⁻⁴ mmol zirconium). Therefore, the atomic ratio of zirconium to aluminium was 6.66 x 10⁻⁴ : 2.2, or approximately 1 : 3303.

There is no teaching anywhere in EP-A-69 951 of the detailed molecular structures of the polymers obtained and, furthermore, no suggestion therein that polymers derived from higher olefins, i.e. those containing 6 or more carbon atoms, would or should have similar structures to those derived from lower olefins such as propylene.

In WO 89/12662 it is disclosed that a characteristic of the molecular structures of 1-alkene oligomers that has been found to correlate very well with improved lubricant properties in commercial lubricants is the ratio of methyl to methylene groups (branch ratio) in the oligomer. In particular, viscosity index (VI) has been found to increase with lower branch ratio.

WO 89/12662 describes liquid lubricant compositions and a process for their preparation, which comprise C₃₀-C₁₃₀₀ hydrocarbons, the compositions having a (low) branch ratio of less than 0.19, weight average molecular weight between 300 and 45,000, number average molecular weight between 300 and 18,000, molecular weight distribution between 1 and 5 and pour point below -15°C.

The liquid lubricant compositions are prepared by a process which comprises oligomerising an olefin containing from 2 to about 20 carbon atoms, preferably an alpha-olefin, e.g. a C₇-C₁₆, preferably C₈-C₁₄, alpha-olefin, under oligomerisation conditions in contact with a supported and reduced valence state metal oxide catalyst from Group VI B of the IUPAC Periodic Table, preferably chromium oxide. The process is carried out at a temperature of 90 to 250°C in order to produce oligomers having molecular weights within the above-specified ranges. This temperature range is stated (Page 18, line 32 to Page 19, line 1) to be higher than the temperature suitable to produce high molecular weight polyalpha-olefins.

At Page 12, line 27 to Page 13, line 1 of WO 89/12662, it is stated that, in general, the novel oligomers have a regular head-to-tail structure: 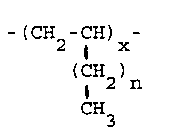
where n is 3 to 17, with some head-to-head connections.

It has now surprisingly been found that by using certain Group IV A catalyst compositions, it is possible to prepare liquid organic compounds from higher olefins in good yield that have a low polymerisation degree and that also have very highly regular head-to-tail structure, which are suitable as base materials for lubricants.

The invention may be defined as relating to a process for the preparation of liquid organic compounds suitable as base materials for lubricants, comprising contacting one or more alpha-olefins containing 8 to 20, preferably 8 to 18, more preferably 8 to 16, still more preferably 8 to 14, and especially 8 to 12, carbon atoms per molecule, under oligomerisation conditions with a catalyst composition based on
a) a Group IV A metal compound of the general formula (Cp)₂ MeX₂, wherein Cp represents a cyclopentadienyl group, Me represents a Group IV A metal and each X independently represents a moiety selected from the group consisting of hydrocarbyl groups, hydrocarboxy groups, hydrocarbamido groups, each of which may be optionally substituted, hydrogen atoms and halogen atoms and
b) a substantially non-coordinating anion source; and optionally subjecting the oligomers formed to an addition reaction to reduce their olefinic unsaturation.

In order to minimize the number of branching groups in the molecules of the oligomeric products, it is preferred to use substantially linear alpha-olefins as starting materials.

Linear alpha-olefins with 8 to 20 carbon atoms per molecule are readily available from processes for the oligomerisation of ethene according to the "Aufbau" -principle. It may be feasible to select the ethene-oligomerisation conditions such that predominantly products within the desired range of 8 to 20 carbon atoms per molecule are formed. However, in most existing ethene-oligomerisation processes a wide range of olefins is formed, viz. olefins having from 4 to 30 carbon atoms per molecule. By conventional separation techniques, such as fractional distillation, olefins within the desired range of 8 to 20 and preferably within the range of 8 to 14 carbon atoms per molecule can easily be recovered.

A convenient process for the catalytic oligomerisation of ethene is described in US Patent No.3647915.

In the process of the invention the alpha-olefins containing 8 to 20, and preferably 8 to 14 carbon atoms per molecule are converted to products, typically having a number-average molecular weight in the range of 400 to 3000, preferably in the range of 400 to 1000 and most preferably in the range of 400 to 700.

Products having higher molecular weights, e.g. number-average molecular weights of 6000 or more, generally are less suitable as base materials for lubricants and hence the oligomerisation conditions are preferably selected such that the molecules of the obtained product are predominantly derived from 2 to 8 monomeric units. For certain applications, dimers are suitable which are, preferably, derived from monomers having carbon numbers in the higher region of the above mentioned carbon range.

In the Group IV A metal compounds participating in the catalyst compositions of the invention, the Group IV A metal is preferably zirconium or hafnium. Zirconium compounds are in particular preferred.

In the present specification and claims, the Group IV A metal compounds are represented by the general formula (Cp)₂ MeX₂. In the compounds of this formula the Group IV A metal (Me) is linked to two cyclopentadienyl groups (Cp) and to two moieties X which may be the same or different and are selected from optionally substituted hydrocarbyl, hydrocarboxy and hydrocarbamido groups, hydrogen atoms and halogen atoms, e.g. bromine, and preferably chlorine, atoms.

Examples of hydrocarbyl groups include alkyl, aralkyl, cycloalkyl, aryl and alkaryl groups. In this specification, unless otherwise stated, an alkyl group or an alkyl moiety in an aralkyl or alkaryl group may be linear or branched and preferably contains up to 20, more preferably up to 12 and especially up to 10 carbon atoms. Particularly preferred alkyl groups are methyl, ethyl, propyl, hexyl and nonyl groups. An aryl group may be any aromatic group, especially a phenyl or naphthyl group. An aralkyl group is an alkyl group substituted by an aryl group, such as a phenylmethyl or phenylethyl group. An alkaryl group is an aryl group substituted by one or more, preferably one to two, alkyl groups. A cycloalkyl group may contain from 3 to 8, preferably 3 to 6, carbon atoms, e.g. a cyclopentyl or cyclohexyl group.

Examples of hydrocarboxy groups include alkoxy groups, preferably C₁-C₂₀, more preferably C₁-C₁₀, alkoxy groups such as methoxy, ethoxy and butoxy groups, and aryloxy groups, e.g. a phenoxy group.

Examples of hydrocarbamido groups include acetamido and propionomido groups.

If desired, the hydrocarbyl, hydrocarboxy and hydrocarbamido groups may each be optionally substituted by one or more, preferably one or two, substituents selected from halogen atoms (e.g. chlorine atoms), nitro, hydroxyl, cycloalkyl, alkoxy, haloalkoxy, amino, dialkylamino, formyl, alkoxycarbonyl, carboxyl, alkanoyl, alkylthio, alkylsulphinyl, alkylsulphonyl, carbamoyl, alkylamido, aryl, alkaryl and heterocyclyl groups. When any of the foregoing substituents contain an alkyl moiety, this may be linear or branched and may contain up to 12, preferably up to 6, and especially up to 4, carbon atoms. A heterocyclyl group may be any saturated or unsaturated ring system, e.g. a C₅-C₇ ring system, containing at least one heteroatom selected from oxygen, nitrogen and sulphur, 5- and 6- membered rings being especially preferred, e.g. a tetrahydrofuranyl, furanyl, piperidinyl, pyridinyl, tetrahydrothiophenyl or thiophenyl (thienyl) group.

However, for practical reasons, the preparation of compounds of formula (Cp)₂MeX₂ wherein each X independently represents a moiety selected from the group consisting of unsubstituted hydrocarbyl groups, unsubstituted hydrocarboxy groups, unsubstituted hydrocarbamido groups, hydrogen atoms and halogen atoms, is preferred.

Preferably, each X independently represents a hydrocarbyl group, a hydrocarboxy group, e.g. a lower alkoxy group such as a methoxy or ethoxy group, or a halogen atom.

More preferably, each X independently represents a hydrocarbyl group, e.g. an alkyl group such as a C₁-C₄ alkyl group, or a halogen atom, e.g. chlorine atom.

Compounds of formula (Cp)₂MeX₂ in which each X independently represents a methyl group or a chlorine atom are particularly preferred.

The Group IV A metal compounds can be prepared by methods known per se. For example, bis(cyclopentadienyl) zirconium dichloride may be prepared by reaction of cyclopentadienyl lithium with zirconium tetrachloride and by subsequent reaction with methyl lithium, bis(cyclopentadienyl) zirconium dimethyl may be obtained.

The catalyst compositions used in the process of the invention are further based on a source of substantially non-coordinating anions, i.e. a source of anions which do not, or only to a minor extent, coordinate with the Group IV A metal atom. Examples of substantially non-coordinating anion sources include sources of carborane anions, such as B₁₁C₁₂H₁₂ anions and aluminoxanes. Aluminoxanes are preferred anion sources.

Aluminoxanes are well known polymeric aluminium compounds which can be represented by the general formulae (R-Al-O)_{q}, which represents a cyclic compound, and R(R-Al-O)_{q}-AlR₂, which represents a linear compound. More complicated structures may exist as well. In these general formulae R represents an alkyl group, preferably having in the range of from 1 to 5 carbon atoms, such as methyl, ethyl, iso-butyl or iso-propyl and q is an integer of from 1 to 100, especially q is in the range of from 5 to 20. Also very effective is a mixture of methyl and isobutyl aluminoxane. Most preferably R is methyl, so that preferably the aluminoxane comprises a methylaluminoxane. The aluminoxanes are suitably prepared by reacting water with trialkylaluminium compounds by methods known in the art. A mixture of linear and cyclic compounds is usually obtained.

The molar ratio of the aluminoxane to the Group IV A metal compound may vary between wide ranges. Conveniently, the molar ratio of the aluminoxane to the Group IV A metal compound is within the range of from 50:1 to 2000:1, preferably from 300:1 to 1000:1, calculated as gram atom aluminium per gram atom of Group IV A metal.

The catalyst composition of the present invention may be prepared from the Group IV A metal compound and the anion source prior to the contacting with the alpha-olefin(s) to be oligomerised or they may be prepared in situ, i.e. in the presence of the alpha-olefins. It is preferred to prepare the catalyst composition by mixing together the two components in solution in a solvent such as toluene to form a liquid catalyst system.

The amount of catalyst composition, used in the process of the invention is usually selected such that per mole of alpha-olefin to be oligomerised, from 10⁻² to 10⁻⁷ gram atom of Group IV A metal is supplied.

The oligomerisation reaction is conveniently carried out at a temperature in the range of 0 to 100 °C, preferably in the range of 10 to 60 °C. Most preferably, a reaction temperature in the range of 20 to 45 °C is selected.

Although not strictly necessary, the process of the invention is preferably carried out in the presence of an inert liquid diluent. Suitably a diluent is selected which can also be used as the solvent for the catalyst composition.

Examples of suitable diluents are alkylbenzenes, such as toluene, chlorinated hydrocarbons, such as dichloromethane and dichloroethane.

The process can be carried out in batch or in continuous operation.

Preferably, the oligomerisation reaction is performed in the substantial absence of air or moisture.

As explained above, suitable base materials for lubricants have to fulfil certain requirements, in particular as regards their structure, such as degree of branching, and their molecular weight.

A further requirement is the substantial absence of olefinic unsaturation in potential lubricant components. Olefinic unsaturation is especially undesirable in compounds of relatively low molecular weight, as the stability of these compounds under oxidative conditions is usually limited. The presence of significant olefinic unsaturation in the molecules of compounds intended for use in lubricant compositions, would result in the formation of decomposition products during use, by oxidation of double bonds, which would be at the expense of the lubricating properties of the compositions.

Hence, it is preferred to substantially remove any olefinic unsaturation in the compounds prepared according to the invention, before they are used as components for lubricants. This is effected by subjecting the oligomers formed to an addition reaction.

A convenient method for achieving this, comprises addition of hydrogen by subjecting the oligomeric product to a hydrogenation treatment. Suitably the hydrogenation treatment is performed catalytically, in particular in the presence of conventional catalysts, such as nickel -, platinum- or palladium-containing catalysts.

In principle, the saturation of double bonds in the oligomeric molecules may be achieved in situ, viz. by adding hydrogen to the reaction mixture during the oligomerisation reaction. The supply of larger or smaller quantitites of hydrogen to the mixture also allows a control of the molecular weight of the formed oligomeric products. However, as the presence of hydrogen during the oligomerisation reaction would result in the hydrogenation of otherwise interesting unsaturated lower-boiling by-products, it is preferred to perform the hydrogenation in a separate step, subsequent to the oligomerisation reaction.

Other methods for substantially removing olefinic unsaturation from the oligomeric products, involve addition reactions wherein functional groups are introduced in the oligomeric compounds in one or more reaction steps, following the oligomerisation reaction. In this manner, in addition to saturating olefinic double bonds, the introduction of selected functional groups can improve the properties of the compounds, required for their use in lubricants.

For example, in a functionalisation step, following the oligomerisation reaction, a thiol may be added to the double bond of an oligomer, thus introducing a mercapto group in the molecule. Epoxy groups may be introduced by reaction with hydrogen peroxide, or a substituted phenyl group by reaction with a substituted benzene. Furthermore, ester groups may be introduced by radical reaction with thioglycolic acid in the presence, e.g. of lauryl peroxide, followed by reaction with an alcohol, e.g. hexanol or 2-ethylhexanol.

Preferred functional groups are succinic acid, succinic acid ester and succinimide groups. Such groups may conveniently be introduced by reacting the oligomeric product with maleic anhydride and, optionally, in a second functionalisation step, with an alcohol or amine, e.g. a polyamine.

Preferably, the number of functional groups, introduced per molecule of oligomer product is in the range from 0.7 to 1. Any remaining unsaturation may be removed by a hydrogenation treatment, as indicated above.

The liquid organic compounds prepared according to the invention, which compounds may contain functional groups and from which any olefinic unsaturation has been substantially removed, are eminently suitable as participants in lubricating compositions, but also as dispersant luboil components or additives. These compositions generally comprise a major amount of a lubricating oil and a minor amount of one or more additives such as dispersant additives, detergents, VI improvers, anti-oxidants and friction-reducing agents.

The invention will be further understood from the following illustrative examples.

### Example 1

### a) Oligomerisation of olefins

In an inert atmosphere a solution of 7.5 g of commercially available methylaluminoxane, dissolved in 80 mL of toluene was introduced in a 3-L reaction vessel equipped with a mechanical stirrer. To this solution was added 600 mL of dry toluene, 360 mL (274 g) of dry technical 1-dodecene, having a 1-dodecene content of >92% m/m and consisting of the compounds mentioned in Table 1, and 250 mL (180 g) of dry technical 1-octene, having a 1-octene content of >95.5% m/m and consisting of the compounds mentioned in Table 2. In an inert atmosphere the temperature of the mixture was raised to 30°C. An amount of 75 mg of commercially available bis(cyclopentadienyl)zirconium dichloride was dissolved in 10 mL of dry toluene and subsequently the solution was added to the reactor. The reaction temperature was maintained at 30°C. After stirring for 20 hours the reaction mixture was treated at 30°C with 60 mL of water during 1 hour under stirring. After cooling to room temperature the mixture was extracted with 200 mL of a 5% m/m sodium chloride (NaCl) solution in water. The oligomer solution in toluene was separated from the aqueous layer. The aqueous layer was extracted three times with 100 mL of toluene. The toluene extracts were combined and subsequently washed with 200 mL of 5% m/m aqueous sodium chloride (NaCl). The toluene solution was separated and was subsequently dried over anhydrous magnesium sulphate (MgSO₄). The toluene and the unconverted olefins were evaporated at 0.04 mbar (4 Pa) at 110°C. The amount of remaining oligomers was 390 g.

According to gas liquid chromatography (GLC), the conversion of 1-olefins was greater than 90%.

The molecular structure of the oligomerisation product was investigated by ¹H-NMR and ¹³C-NMR spectroscopy. The ¹³C-NMR spectrum showed the presence of two sets of two types of unsaturated carbon atoms, viz. = CH₂ (delta = 109.8, 109.9 ppm for the oligomers; delta = 108.1 ppm for dimer) and -CR= (delta = 147.8 ppm for the oligomers; delta = 148.8 ppm for dimer), thus indicating terminal vinylidene groups of the formula -CR=CH₂ for both the oligomers and the minor amount of dimer (<8% m/m) present in the oligomerisation product. From this spectrum, it could be deduced that at least 95% of the product possessed a vinylidene end-group. This was confirmed by ¹H-NMR. The ¹³C-NMR spectrum showed reasonably well defined peaks for the CH₃, CH₂ and CH carbon atoms along the chain. Thus, the oligomers were all substantially of the formula 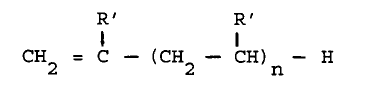
wherein each R' = C₆H₁₃ or C₁₀H₂₁.

The oligomers were introduced into a fractionating column, in order to remove the dimers having less than 24 carbon atoms as well as any lower boiling compounds. The amount of oligomers, recovered as bottom fraction was 279 g.

The number-average molecular weight was 560.

**Table 1**

| Composition of technical 1-dodecene | |
|---|---|
| Carbon distribution, % m/m: | |
| C10 and less | <1 |
| C12 | >97 |
| C14 and greater | <2 |

| Hydrocarbon type, % m/m: | |
|---|---|
| Total n-alpha olefins | >94 |
| C12 alpha olefin | >92 |
| Branched plus internal olefins | <6 |
| Paraffins | <0.2 |

**Table 2**

| Composition of technical 1-octene | |
|---|---|
| Carbon distribution, % m/m: | |
| C6 and less | <0.5 |
| C8 | >99.0 |
| C10 and greater | <0.5 |

| Hydrocarbon type, % m/m: | |
|---|---|
| Total n-alpha olefins | >96.5 |
| C8 alpha olefin | >95.5 |
| Branched plus internal olefins | <3.5 |
| Paraffins | <0.1 |

### b) Hydrogenation of oligomers

A 500-mL Hastelloy autoclave equipped with a mechanical stirrer, was charged with 80 g of olefin oligomers, prepared according to Example 1a) and 75 mL of 1,4-dioxane. An amount of 16 g of a finely divided catalyst, comprising 5% m/m palladium on a charcoal support was added. The autoclave was pressurized with H₂ to 60 bar (6 MPa) and under stirring the temperature was raised to 150°C. During this heating-up period, which took about 2 hours, >95% of the theoretical amount of hydrogen was consumed. After the reaction mixture had been stirred at 150°C for another 14 hours, it was cooled to room temperature and was subsequently filtered. The catalyst was washed with 75 mL of 1,4-dioxane and subsequently the volatiles of the combined filtrate were removed in a rotary evaporator at <5mbar (<500 Pa) at 110°C. The yield of hydrogenated oligomers was 76 g.

As shown by ozone titration, and by ¹H- and ¹³C-NMR spectroscopy the product did not exhibit any significant unsaturation (less than 0.4%).

Samples of the oligomeric product were taken and an evaluation was made of the following physical and performance characteristics:
Kinematic viscosity (using ASTM D445);
Viscosity index (using ASTM D2270);
Pour point (using ASTM D97);
Oxidation stability (using DSC, as indicated below*);
Volatility (using thermogravimetric method TGA-IP 393/91).
Molecular weight distribution, defined as the ratio of weight average molecular weight (M_{w}) to number average molecular weight (Mₙ) (M_{w}/Mₙ) **;
* Differential Scanning Calorimetry (DSC)
DSC monitors the differential power supplied to a pair of heaters as a function of time or temperature, as the sample and a suitable reference material are subjected to the following temperature programme: Initial test temperature 40 °C, final temperature 350 °C. Heating rate 20 °C/minute. DSC cell atmosphere of 15 psi (103.4 kPa) gauge pressure of oxygen. Purge flow rate of 60± 10 mL/min.
** Molecular Weight Distribution (MWD)
MWD was determined by high temperature - programmed (35-440°C) gas chromatography, using a flame ionisation detector at 450°C (carrier gas: helium, 10 mL/min). The analysis was carried out by on-column injection (injector temp.: 25°C; sample: 1 microlitre of 1/1000 solution in carbon tetrachloride) on a 6AQ5/HT5 (siloxane-carborane copolymer) capillary column (6 m x 0.53 mm; film thickness: 0.10 micrometre) purchased from Scientific Glass Engineering Inc., USA (catalogue number 051580).

During the sample run, the control software processes the signals such that they represent the differential heat flow between the sample and the reference material.

Since oxidation of lubricating oils is generally exothermic, DSC is used to detect the heat evolved during oxidation.
Measurements are made with 2.0 ± 0.05 mg samples in a prepared open aluminium pan, an identical empty pan being used as reference. Extrapolated onset temperatures are determined and given as the nearest whole number (°C).

Branch ratio, defined as in WO 89/12662 as the ratio of wt. fraction of methyl group (Z) to 1-(wt. fraction of methyl group), i.e. 1-Z, (Z/1-Z) (determined by ¹³C-NMR).

The evaluation results are shown in Table 3.

### Example 2

### a) Oligomerisation of olefins

Similarly to Example 1a) an oligomerisation was carried out using 50 mL of an equimolar mixture of technical 1-dodecene, having the composition mentioned in Table 1 and technical 1-octene, having the composition mentioned in Table 2. The resulting oligomer was worked-up along the lines mentioned in Example 1a), with the difference that no fractionation was carried out to remove the dimers having less than 24 carbon atoms. Analysis by ¹H- and ¹³C-NMR spectroscopy showed that >95% of the end-groups of the oligomers were vinylidene groups. According to GLC, the conversion of 1-olefins was greater than 90%.

The number-average molecular weight was 500.

### b) Hydrogenation of oligomers

The oligomers prepared according to Example 2a) were hydrogenated in a similar manner as described in Example 1b). As shown by ozone titration and by ¹H- and ¹³C-NMR spectroscopy the remaining unsaturation of the hydrogenated product was less than 1 %.

An evaluation of performance properties of the product was made as indicated in Example 1b). The evaluation results are shown in Table 3.

### Example 3

### a) Oligomerisation of olefins

Along the lines of Example 2a) 50 mL of technical 1-octene, having the composition mentioned in Table 2, was oligomerised. Analysis by ¹H- and ¹³C-NMR spectroscopy showed that >95% of the end-groups of the oligomers were vinylidene groups. According to GLC, the conversion of 1-octene was greater than 90%.

The number-average molecular weight was 450.

### b) Hydrogenation of oligomers

The oligomers prepared according to Example 3a) were hydrogenated in a similar manner as described in Example 1b). As shown by ozone titration and by ¹H- and ¹³C-NMR spectroscopy the remaining unsaturation of the hydrogenated product was less than 1 %.

An evaluation of performance properties of the product was made as indicated in Example 1b). The evaluation results are shown in Table 3.

### Example 4

### a) Oligomerisation of olefins

Along the lines of Example 2a) 50 mL of technical 1-dodecene, having the composition mentioned in Table 1, was oligomerised. Analysis by ¹H- and ¹³C-NMR spectroscopy showed that >95% of the end-groups of the oligomers were vinylidene groups. According to GLC, the conversion of 1-dodecene was greater than 90%. The number-average molecular weight was 650.

### b) Hydrogenation of oligomers

The oligomers prepared according to Example 4a) were hydrogenated in a similar manner as described in Example 1b). As shown by ozone titration and by ¹H- and ¹³C-NMR spectroscopy the remaining unsaturation of the hydrogenated product was less than 1%.

An evaluation of performance properties of the product was made as indicated in Example 1b). The evaluation results are shown in Table 3.

### Comparative Examples A and B (not according to the invention)

The physical and performance characteristics of two commercial products were evaluated in the manner, as indicated in Example 1b). The commercial samples were:
A. Mobil SHF 61 (hydrogenated poly alpha olefins);
B. XHVI 5 (Trade mark).

The evaluation results are shown in Table 3.

**Table 3**

| Ex | Mₙ | M_{w}/ Mₙ | Branch ratio | Vₖ100 mm²/s | VI | Pour pt °C | DSC onset °C (oxid. stab.) | TGA loss up to 262°C % m/m (volatility) |
|---|---|---|---|---|---|---|---|---|
| 1 | 560 | 1.2 | 0.16 | 6.53 | 157 | -39 | 213 | 9.5 |
| 2 | 500 | 1.4 | 0.17 | 5.93 | 177 | -54 | 208 | >27 |
| 3 | 450 | 1.7 | 0.22 | 6.72 | 166 | -57 | 201 | >28 |
| 4 | 650 | 1.5 | 0.14 | 6.32 | 202 | -27 | 209 | 19 |
| A | | - | 0.24 | 5.63 | 136 | -62 | 210 | 10 |
| B | | - | 0.23 | 5.49 | 153 | -18 | 206 | 11.9 |

As can be seen from the evaluation results the hydrogenated oligomers according to the invention exhibit excellent viscosity index values. As regards oxidation stability they are on a par with the commercial products. Their pour points are consistent with those of product A and significantly better than those of product B. If for certain applications the volatility is of particular importance, this requirement can easily be met, as shown by Example 1.

## Claims

1. A process for the preparation of liquid organic compounds, suitable as base materials for lubricants, comprising contacting one of more alpha-olefins containing 8 to 20 carbon atoms per molecule, under oligomerisation conditions with a catalyst composition based on
a) a Group IV A metal compound of the general formula (Cp)₂ MeX₂, wherein Cp represents a cyclopentadienyl group, Me represents a Group IV A metal and each X independently represents a moiety selected from the group consisting of hydrocarbyl groups, hydrocarboxy groups, hydrocarbamido groups, each of which may be optionally substituted, hydrogen atoms and halogen atoms and
b) a substantially non-coordinating anion source; and optionally subjecting the oligomers formed to an addition reaction to reduce their olefinic unsaturation.

2. A process according to claim 1, wherein as starting material, one or more linear alpha-olefins with 8 to 20 carbon atoms per molecule are used.

3. A process according to claim 1 or 2, wherein one or more alpha-olefins with 8 to 14 carbon atoms per molecule are used.

4. A process according to any one of claims 1 to 3, wherein Me represents zirconium or hafnium.

5. A process according to any one of the preceding claims, wherein each X independently represents an alkyl group or a chlorine atom.

6. A process according to any one of the preceding claims, wherein the substantially non-coordinating anion source is an aluminoxane.

7. A process according to claim 6, wherein the aluminoxane is methylaluminoxane.

8. A process according to claim 7 or 8, wherein the molar ratio of aluminoxane to Group IV A metal compound is in the range from 300:1 to 1000:1, calculated as gram atom aluminium per gram atom Group IV A metal.

9. A process according to any one of the preceding claims, wherein oligomerisation is carried out at a temperature in the range from 10 to 60 °C.

10. A process according to any one of the preceding claims, wherein the oligomers formed are subjected to a hydrogenation treatment.
